# EUROPEAN PATENT SPECIFICATION

(11) **EP 2 245 933 B1**
(45) Date of publication and mention of the grant of the patent: **16.12.2015**
(21) Application number: 09708150.9
(22) Date of filing: 06.02.2009
(51) Int. Cl.: A01N 37/06, A01G 7/06, A01N 25/04, A01N 61/00, A01P 21/00

(54) **AGENT FOR PREVENTING POLLEN DISPERSAL**
POLLENVERBREITUNGSHEMMER
AGENT POUR EMPÊCHER LA DISPERSION DE POLLEN

(30) Priority: 08.02.2008 JP 2008028253; 08.02.2008 JP 2008028254; 18.02.2008 JP 2008036632; 18.02.2008 JP 2008036633
(43) Date of publication of application: 03.11.2010
(73) Proprietor: NOF Corporation, Shibuya-ku Tokyo 150-6019 (JP)
(72) Inventor: KOSHIO, Kaihei, Tokyo 156-8502 (JP); OHIKE, Hirokazu, Amagasaki-shi Hyogo 660-0095 (JP); SHIMADA, Masahiko, Amagasaki-shi Hyogo 660-0095 (JP); SHIINO, Daijiro, Amagasaki-shi Hyogo 660-0095 (JP); TSURUOKA, Kuniaki, Amagasaki-shi Hyogo 660-0095 (JP); YAMANAKA, Aiko, Amagasaki-shi Hyogo 660-0095 (JP)
(74) Representative: Von Kreisler Selting Werner - Partnerschaft von Patentanwälten und Rechtsanwälten mbB
(86) International application number: PCT/JP2009/052101
(87) International publication number: WO 2009/099212

(56) References cited:
- WO-A2-01/28961
- JP-A- 4 099 416
- JP-A- 5 238 902
- JP-A- 7 053 307
- JP-A- 10 098 941
- JP-A- 2005 008 576
- DATABASE CA [Online] CHEMICAL ABSTRACTS SERVICE, COLUMBUS, OHIO, US; 2001, GILCHRIST, HELEN S.: "The preparation and constitution of synthetic fats containing a carbohydrate chain", XP002686274, retrieved from STN Database accession no. 17:22976 & GILCHRIST, HELEN S.: "The preparation and constitution of synthetic fats containing a carbohydrate chain", REPT. BRIT. ASSOC. ADVANCEMENT SCI. ( 1922 ) 357, 1922,
- DATABASE CA [Online] CHEMICAL ABSTRACTS SERVICE, COLUMBUS, OHIO, US; 1988, HATANAKA, CHIAKI ET AL: "Enzymatic synthesis of sucrose ester of fatty acid by Candida lipase", XP002686275, retrieved from STN Database accession no. 109:53149 & HATANAKA, CHIAKI ET AL: "Enzymatic synthesis of sucrose ester of fatty acid by Candida lipase", KITAKYUSHU KOGYO KOTO SENMON GAKKO KENKYU HOKOKU ( 1988 ), 21, 59-63 CODEN: KKKHDI; ISSN: 0285-5283, 1988,
- KOSHIO K. ET AL.: 'Field Trial of Salad Oil Application for the Induction of Male Flower Browning of Cryptomeria Japonica' JOURNAL OF FOREST RESEARCH vol. 5, no. 2, 16 May 2000, pages 77 - 80, XP008138932

## Description

### Technical Field

The present invention relates to the use of an agent for suppressing pollen dispersal, which suppresses dispersion of pollen from plants.

### Background Art

Of the pollens present in the male organ of plants, anemophilous pollen is dispersed in the air by being carried on the wind, and attaches to the ovule for pollination. When people inhale the pollen, a severe symptom called pollinosis is sometime developed. Particularly, in pollinosis due to pollen of Cryptomeria, Chamaecyparis, Ambrosia and the like, allergic conditions of the eye and nose are developed. The number of people suffering from the pollinosis tends to increase every year.

As a solution for such damages by pollen, administration of antihistamine agents, adrenal cortex hormone and the like, and use of eye drops, wearing masks and the like can be mentioned. The most effective solution is suppressing dispersal of pollen into the air.

As a measure for suppressing dispersal of pollen into the air, cutting down causative trees and plants and weeding can be mentioned. However, cutting down trees and the like are problematic in that they not only require enormous labor but also cause an adverse influence such as destruction of natural environment and the like.

As a means for suppressing dispersal of pollen other than cutting down trees, spraying or applying a certain kind of agent onto the male organ has been proposed. As examples of such agent, vegetable oils and fats containing oleic acid or linoleic acid as a main component (e.g., patent document 1) and those containing sodium oleate (e.g., patent document 2) as an active ingredient have been proposed.

However, since these agents show a comparatively slow efficacy on male flower buds, they are defective in that the timing of spraying of the agent is limited to August when male flower buds are in the initial period of bud differentiation process.
patent document 1: JP-B-8-762
patent document 2: JP-B-2890162

### Disclosure of the Invention

### Problems to be Solved by the Invention

Therefore, the present invention aims to provide an agent for suppressing pollen dispersal, which has a sharp action on the male organ and does not influence the trunk, branches and leaves of trees.

### Means of Solving the Problems

The present inventors have conducted intensive studies and found that the above-mentioned object can be achieved, which resulted in the completion of the present invention. Accordingly, the present invention is directed to the
[1] use of an agent comprising, as an active ingredient, an oleic acid derivative or linoleic acid derivative represented by the following formula (I) 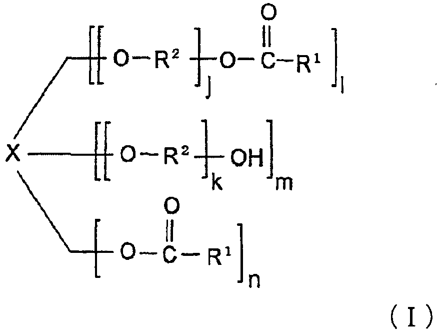 wherein R¹ are residues of an oleic acid ester or a linoleic acid ester, which may be of the same kind or different kinds, O-R² are oxyalkylene groups having a carbon number of 2 to 4, which may be of one kind of two or more kinds, and when they are of two or more kinds, they may be block adducts or random adducts;
   when j+k is an integer of one or more and X is monovalent, then X is a hydrogen atom, a hydrocarbon group having a carbon number of 20 or below or an acyl group having a carbon number of 20 or below, j is an integer of 1 - 100, 1=1 and m+n=0;
   when j+k is an integer of one or more and X is a polyvalent organic group, then j and k are each an integer of 1 - 100, 1+m+n is an integer of two or more and 1+n is an integer of one or more; when j and k are 0 and X is a polyvalent organic group having a carbon number of 3, then m+n is 3, 1 is 0 and n and m is 1 or 2; and
   when j and k are 0 and X is a polyvalent organic group having a carbon number of 4 or more, then 1 is 0, n is an integer of one or more, and m+n is an integer of two or more,
   for suppressing pollen dispersal.
[2] The use according to item 1, wherein the oleic acid derivative or linoleic acid derivative is polyoxyethylene oleic acid ester or polyoxyethylene linoleic acid ester, or polyoxyethylene sorbitan oleic acid ester or polyoxyethylene sorbitan linoleic acid ester, or polyoxyethylene sorbitol oleic acid ester or polyoxyethylene sorbitol linoleic acid ester.
[3] The use according to item 2, wherein the agent for suppressing pollen dispersal is an emulsion comprising the oleic acid derivative or linoleic acid derivative, which has an HLB of not more than 12, in a proportion of 0.5 - 30 wt%.
[4] The use according to item 2, wherein the agent for suppressing pollen dispersal is an aqueous solution comprising the oleic acid derivative or linoleic acid derivative, which has an HLB of not less than 12, in a proportion of 0.5 wt% or above.
[5] The use according to item 1, wherein the oleic acid derivative or linoleic acid derivative is comprised of a partial ester of oleic acid or linoleic acid and glycerol.
[6] The use according to item 5, wherein the agent for suppressing pollen dispersal is an emulsion comprising a partial ester of oleic acid or linoleic acid and glycerol in a proportion of 0.5 - 30 wt%.
[7] The use according to item 1, wherein the oleic acid derivative or linoleic acid derivative is comprised of an ester of oleic acid or linoleic acid and a polyvalent alcohol having a carbon number of not less than 4.
[8] The use according to item 7, wherein the agent for suppressing pollen dispersal is an emulsion comprising an ester of oleic acid or linoleic acid and a polyvalent alcohol having a carbon number of not less than 4 in a proportion of 0.5 - 30 wt%.
[9] The use according to item 1, wherein the oleic acid derivative or linoleic acid derivative is comprised of an ester of oleic acid or linoleic acid and an alcohol having a sugar backbone.
[10] The use according to item 9, wherein the agent for suppressing pollen dispersal is an emulsion comprising an ester of oleic acid or linoleic acid and an alcohol having a sugar backbone in a proportion of 0.5 - 30 wt%.

### Effect of the Invention

The present invention is directed to the use of an agent for suppressing pollen dispersal, which has a sharp action on the male organ and does not influence the trunk, branches and leaves of trees. In addition, the form of an emulsion produced by diluting the agent for suppressing pollen dispersal by adding water affords high solution stability.

### Best Mode for Carrying out the Invention

The agent for use in suppressing pollen dispersal is explained in the following.

The present invention is the use of an agent for suppressing pollen dispersal, comprising, as an active ingredient, an oleic acid derivative or linoleic acid derivative represented by the following formula (I)

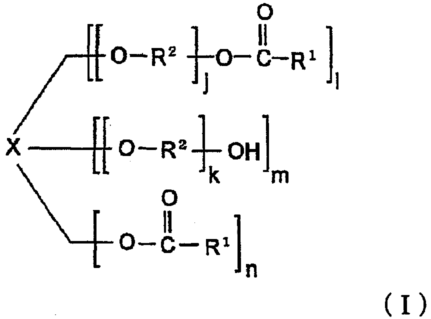

In the aforementioned formula (I), R¹ are residues of an oleic acid ester or linoleic acid ester, which may be of the same kind or different kinds. Fatty acid esters other than oleic acid and linoleic acid are not preferable, since some show an unsharp action on the male organ, and some influence the trunk, branches and leaves of trees, even though the action on the male organ is sharp.

In the aforementioned formula (I), O-R² are oxyalkylene groups having a carbon number of 2 to 4, which may be of one kind of two or more kinds, and when they are of two or more kinds, they may be block adducts or random adducts. O-R² are preferably oxyalkylene groups having a carbon number of 2 to 4. A compound having a carbon number of 1 is (poly)acetal having formaldehyde as a starting material, and is not preferable since the kind of the synthesis starting material is different, and develops formaldehyde as a decomposition product. In addition, alkylene oxide having a carbon number of not less than 5 is not preferable since the supplyability and reactivity of the starting material are low. Moreover, the oxyalkylene group represented by O-R² may be of one kind of two or more kinds, and when they are of two or more kinds, they may be block adducts or random adducts. When an ethylene oxide group having a carbon number of 2 is selected, HLB becomes higher, and then water-solubility can be imparted. On the other hand, as the carbon number increases, the oil-solubility becomes high and HLB becomes low, and therefore, the carbon number can be appropriately selected.

In the aforementioned formula (I), when j+k is an integer of one or more and X is monovalent, then X is a hydrogen atom, a hydrocarbon group having a carbon number of 20 or below, or an acyl group having a carbon number of 20 or below, j is an integer of 1 - 100, 1=1 and m+n=0. When X is a monovalent organic group, X is a hydrocarbon group having a carbon number of 20 or below, or an acyl group having a carbon number of 20 or below. When the carbon number exceeds 20, supplyability becomes low even though the property does not change and unpreferably becomes expensive. j is an integer of 1 - 100. When it exceeds 100, the number of the oleic acid ester group or linoleic acid ester group becomes relatively small, and the action on the male organ is unpreferably lost. When X is monovalent, 1=1 and m+n=0.

In the aforementioned formula (I), when j+k is an integer of one or more and X is a polyvalent organic group, then j and k are each an integer of 1 - 100, 1+m+n is an integer of two or more, and 1+n is an integer of one or more. When X is a polyvalent organic group, X is preferably a residue of an existing alcohol compound without a hydroxyl group, since it shows high supplyability. j and k are each an integer of 1 - 100. When they exceed 100, the number of oleic acid ester group or linoleic acid ester group becomes relatively small to lose action on the male organ, which is not preferable. When X is a polyvalent organic group, 1+m+n is an integer of two or more, and 1+n is an integer of one or more. When l+n=0, the oleic acid ester group and linoleic acid ester group are absent and the action on the male organ is unpreferably lost. While l+m+n may exceed 1000, it is not practical since the viscosity becomes high.

In the aforementioned formula (I), when j and k are 0 and X is a polyvalent organic group having a carbon number of 3, m+n is 3, 1 is 0 and n and m are each 1 or 2. When X is a polyvalent organic group having a carbon number of 3, the aforementioned formula (I) is preferably a partial ester of oleic acid or linoleic acid and glycerol. A partial ester of fatty acid other than oleic acid and linoleic acid and glycerol is not preferable, since it may show an unsharp action on the male organ, or may influence the trunk, branches and leaves of trees, even though the action on the male organ is sharp.

In the aforementioned formula (I), when j and k are each 0 and X is a polyvalent organic group having a carbon number of 4 or more, 1 is 0, n is an integer of one or more and m+n is an integer of two or more. When X is a polyvalent organic group having a carbon number of 4 or more, the aforementioned formula (I) is preferably an ester of oleic acid or linoleic acid and a polyvalent alcohol having a carbon number of 4 or more, more preferably, an ester of oleic acid or linoleic acid and an alcohol having a sugar backbone. An ester of fatty acid other than oleic acid and linoleic acid and a polyvalent alcohol having a carbon number of 4 or more is not preferable, since it may show an unsharp action on the male organ, or may influence the trunk, branches and leaves of trees, even though the action on the male organ is sharp.

For production of the aforementioned formula (I), when j+k is an integer of one or more and X is a hydrogen atom, a method including directly reacting oleic acid or linoleic acid with alkylene oxide is preferable, and when X is a hydrocarbon group having a carbon number of 20 or below, a method including reacting the corresponding alcohol with alkylene oxide, and adding oleic acid or linoleic acid to perform a general dehydrating reaction is preferable. On the other hand, when X is an acyl group having a carbon number of 20 or below, a method including reacting the corresponding carboxylic acid compound with alkylene oxide, and adding oleic acid or linoleic acid to perform a general dehydrating reaction, a method including directly reacting oleic acid or linoleic acid with alkylene oxide, and adding the corresponding carboxylic acid compound to perform a general dehydrating reaction, a method including adding polyalkylene glycol to the corresponding carboxylic acid compound and oleic acid or linoleic acid to perform a general dehydrating reaction and the like can be mentioned, and the method can be appropriately selected.

Specific examples of the obtained compound of the aforementioned formula (I) include polyoxyethylene oleic acid ester, polyoxyethylene linoleic acid ester, methoxypolyoxyethylene oleic acid ester, ethoxypolyoxyethylene linoleic acid ester, butyloxypolyoxyethylene oleic acid ester, hexyloxypolyoxyethylene linoleic acid ester, dodecyloxypolyoxyethylene oleic acid ester, lauryloxypolyoxyethylene linoleic acid ester, stearyloxypolyoxyethylene oleic acid ester, oleyloxypolyoxyethylene linoleic acid ester, polyoxyethylene oleic acid acetic acid ester, polyoxyethylene linoleic acid acetic acid ester, polyoxyethylene oleic acid butyric acid ester, polyoxyethylene linoleic acid caproic acid ester, polyoxyethylene oleic acid caprylic acid ester, polyoxyethylene lauric acid linoleic acid ester, polyoxyethylene oleic acid stearic acid ester, polyoxyethylene dilinoleic acid ester, polyoxyethylene-block-polyoxypropylene oleic acid ester, polyoxyethylene-ran-polyoxybutylene linoleic acid ester and the like.

On the other hand, for production of the aforementioned formula (I), when j+k is an integer of one or more and X is a polyvalent organic group, a method including reacting a polyvalent alcohol compound wherein the side chain of X is a hydroxyl group with alkylene oxide, and adding oleic acid or linoleic acid to perform a general dehydrating reaction, and a method including adding oleic acid or linoleic acid to a polyvalent alcohol compound to perform a general dehydrating reaction to give an ester, which is reacted with alkylene oxide can be mentioned, and the method can be appropriately selected.

Specific examples of the obtained compound of the aforementioned formula (I) include polyoxyethylene sorbitan oleic acid ester, polyoxyethylene sorbitan linoleic acid ester, polyoxyethylene sorbitol oleic acid ester, polyoxyethylene sorbitol linoleic acid ester, polyoxyethylene glycerol oleic acid ester, polyoxyethylene glycerol linoleic acid ester, polyoxyethylene diglycerol oleic acid ester, polyoxyethylene triglycerol linoleic acid ester, polyoxyethylene methylglucoside oleic acid ester, polyoxyethylene methylglucoside linoleic acid ester, polyoxyethylene-ran-polyoxypropylene sorbitan oleic acid ester, polyoxyethylene-block-polyoxybutylene sorbitan linoleic acid ester, polyvinyl alcohol-graft-(w-oleinoyloxypolyoxyethylene) and the like.

For production of the aforementioned formula (I), when j and k are each 0 and X is a polyvalent organic group having a carbon number of 3, a trivalent alcohol compound wherein the side chain of X is a hydroxyl group and oleic acid or linoleic acid with an adjusted preparation molar ratio of 1 - 2 relative to 3 hydroxyl groups are subjected to a general dehydrating reaction, whereby it can be obtained.

Specific examples of the obtained compound of the aforementioned formula (I) include glycerol monooleate, glycerol dioletate, glycerol monolinolate and glycerol dilinolate. Here, when the aforementioned formula (I) is a partial ester of oleic acid or linoleic acid and glycerol, it can be obtained by adjusting the preparation molar ratio of fatty acid relative to glycerol. A triglyceride of fatty acid having a carbon number of not less than 8 wherein all three hydroxyl groups of glycerol are esterified is defective in that the timing of spraying of the agent is limited, since it shows a comparatively slow efficacy on male flower buds.

For production of the aforementioned formula (I), when j and k are each 0 and X is a polyvalent organic group having a carbon number of 4 or more, it can be obtained by appropriately adjusting the preparation molar ratios of oleic acid or linoleic acid and a polyvalent alcohol having a carbon number of 4 or more, and subjecting them to a general dehydrating reaction.

Specific examples of the obtained compound of the aforementioned formula (I) include 1,2-butanediol monooleate, 1,2-butanediol dioleate, 1,2-butanediol monolinolate, 1,2-butanediol dilinolate, 1,3-butanediol monooleate, 1,3-butanediol dioleate, 1,3-butanediol monolinolate, 1,3-butanediol dilinolate, 1,4-butanediol monooleate, 1,4-butanediol dioleate, 1,4-butanediol monolinolate, 1,4-butanediol dilinolate, pentaerythritol monooleate, pentaerythritol dioleate, pentaerythritol trioleate, pentaerythritol tetraoleate, pentaerythritol monolinolate, pentaerythritol dilinolate, pentaerythritol trilinolate, pentaerythritol tetralinolate, neopentylglycol, 1,5-pentanediol monooleate, 1,5-pentanediol dioleate, 1,5-pentanediol monolinolate, 1,5-pentanediol dilinolate, trimethylolethane monooleate, trimethylolethane dioleate, rtimethylolethane trioleate, trimethylolethane monolinolate, trimethylolethane dilinolate, trimethylolethane trilinolate, trimethylolpropane monooleate, trimethylolpropane dioleate, trimethylolpropane trioleate, trimethylolpropane monolinolate, trimethylolpropane dilinolate, trimethylolpropane trilinolate, 3-methyl-1,5-pentanediol monooleate, 3-methyl-1,5-pentanediol dioleate, 3-methyl-1,5-pentanediol monolinolate, 3-methyl-1,5-pentanediol dilinolate, 1,2-hexanediol monooleate, 1,2-hexanediol dioleate, 1,2-hexanediol monolinolate, 1,2-hexanediol dilinolate, 1,6-hexanediol monooleate, 1,6-hexanediol dioleate, 1,6-hexanediol monolinolate, 1,6-hexanediol dilinolate, 1,2,6-hexanetriol monooleate, 1,2,6-hexanetriol dioleate, 1,2,6-hexanetriol trioleate, 1,2,6-hexanetriol monolinolate, 1,2,6-hexanetriol dilinolate, 1,2,6-hexanetriol trilinolate, hexyleneglycol monooleate, hexyleneglycol dioleate, hexyleneglycol monolinolate, hexyleneglycol dilinolate, dipropyleneglycol monooleate, dipropyleneglycol dioleate, dipropyleneglycol monolinolate, dipropyleneglycol dilinolate, tripropyleneglycol monooleate, tripropyleneglycol dioleate, tripropyleneglycol monolinolate, tripropyleneglycol dilinolate, 2-butyl-2-ethyl-1,3-propanediol monooleate, 2-butyl-2-ethyl-1,3-propanediol dioleate, 2-butyl-2-ethyl-1,3-propanediol monolinolate, 2-butyl-2-ethyl-1,3-propanediol dilinolate, 2,2'-diethyl-2,2'-(oxydimethyl)bis(propane-1,3-diol) monooleate, 2,2'-diethyl-2,2'-(oxydimethyl)bis(propane-1,3-diol) dioleate, 2,2'-diethyl-2,2'-(oxydimethyl)bis(propane-1,3-diol)trioleate, 2,2'-diethyl-2,2'-(oxydimethyl)bis(propane-1,3-diol)tetraoleate, 2,2'-diethyl-2,2'-(oxydimethyl)bis(propane-1,3-diol) monolinolate, 2,2'-diethyl-2,2'-(oxydimethyl)bis(propane-1,3-diol) dilinolate, 2,2'-diethyl-2,2'-(oxydimethyl)bis(propane-1,3-diol) trilinolate, 2,2'-diethyl-2,2'-(oxydimethyl)bis(propane-1,3-diol) tetralinolate, diglycerol monooleate, diglycerol dioleate, diglycerol trioleate, diglycerol tetraoleate, diglycerol monolinolate, diglycerol dilinolate, diglycerol trilinolate, diglycerol tetralinolate, triglycerol monooleate, triglycerol dioleate, triglycerol trioleate, triglycerol tetraoleate, triglycerol pentaoleate, triglycerol monolinolate, triglycerol dilinolate, triglycerol trilinolate, triglycerol tetralinolate, triglycerol pentalinolate; oleic acid or linoleic acid esters of monosaccharides such as glucose, fructose and the like; oleic acid or linoleic acid esters of oligosaccharides such as sucrose, lactose and the like; oleic acid or linoleic acid esters of sugar derivatives such as sorbitol, mannitol, dulcitol, xylitol, erythritol etc. and the like. In the compound, all the hydroxyl groups in an alcohol having a sugar backbone may be esterified, or some of the hydroxyl groups in an alcohol having a sugar backbone may be partially esterified. These esters can be obtained by adjusting the preparation molar ratio of fatty acid relative to an alcohol having a sugar backbone.

Here, when j and k are each 0, X has a carbon number of not more than 3 and 1+m+n=2, namely, an ester of ethyleneglycol or propyleneglycol and oleic acid or linoleic acid is not preferable, since it influences the trunk, branches and leaves of trees, even though the efficacy on the male organ is sharp.

The oleic acid derivative or linoleic acid derivative used according to the present invention can be diluted when in use by adding water. When the oleic acid derivative and linoleic acid derivative have an HLB of 12 or below, they are mostly hardly water-soluble, and the diluted product by adding water becomes an emulsion. When producing an emulsion, the oleic acid derivative or linoleic acid derivative itself functions as a surfactant, which is effective for formation of emulsion. An emulsion may also be produced by adding a generally-used surfactant.

The concentration of an oleic acid derivative or linoleic acid derivative to be contained in the emulsion is preferably 0.5 - 30 wt%, more preferably 1 - 20 wt%. When it is not less than 20 wt%, the emulsion needs to be used immediately after preparation, since it has low stability. When it is not less than 30 wt%, a stable emulsion cannot be obtained.

When the oleic acid derivative and linoleic acid derivative used according to the present invention have an HLB greater than 12, they are mostly water-soluble, the diluted product by adding water becomes an aqueous solution. The concentration of an oleic acid derivative or linoleic acid derivative to be contained in the aqueous solution is preferably not less than 0.5 wt%, more preferably not less than 1 wt%. When it is less than 0.5 wt%, the amount thereof to be sprayed increases to achieve the effect.

The surfactant to be added to an emulsion may be any as long as it is generally used as an emulsifier, and a non-ionic surfactant is particularly preferable. Examples of the non-ionic surfactant include polyoxyethylene alkyl ether type non-ionic surfactant, polyoxyethylene fatty acid ester type non-ionic surfactant, polyoxyethylene fatty acid sorbitan ester type non-ionic surfactant, polyoxyethylene hydrogenated castor oil type non-ionic surfactant, polyoxyethyleneglycerol fatty acid ester type non-ionic surfactant, polyglycerol fatty acid ester type non-ionic surfactant and the like.

The agent for suppressing pollen dispersal which has been formulated as an emulsion or an aqueous solution in such manner can be, for example, sprayed from a helicopter etc. to suppress dispersal of pollen in a wide area.

The agent for suppressing pollen dispersal used according to the present invention may be sprayed on, for example, Cryptomeria japonica in any period after differentiation of the male flower bud, and can be sprayed over a comparatively long period of from August when male flower buds are in the initial period of bud differentiation process to October - November when the pollen grains are in the formation period, whereby the dispersal of pollen in the next spring can be effectively suppressed. Examples

The present invention is explained in more detail in the following by referring to Examples .

### Evaluation 1 (immersion test of Cryptomeria japonica)

First, as agents to be used for an immersion test of Cryptomeria japonica, fatty acid derivative, fatty acid, polyoxyalkylene derivative, fats and oils and the like shown in Tables 1 and 2 were prepared. Next, many branches (length about 10 cm) of Cryptomeria japonica with 15 male flower buds on the tip were taken from the same tree of Cryptomeria japonica, and each group (five leaves for one group) was immersed in the agents (100 ml) of Tables 1 and 2. After immersing for about 1 min, the branches were taken out from the liquid and placed in a beaker. Browning changes were inspected visually on the male flower buds and needle leaves after one week. The test was performed using Cryptomeria japonica of August and November. The results of August are shown in Table 3 and the results of November are shown in Table 4.

Sodium oleate, partial ester of stearic acid and glycerol, ester of stearic acid or palmitic acid and polyvalent alcohol, and ester of stearic acid and sorbitol used in Comparative Examples here are solids at room temperature. Thus, sodium oleate was used as an aqueous solution obtained by dissolving in water to an active ingredient concentration of 5%, and the partial ester of stearic acid and glycerol, the ester of stearic acid or palmitic acid and polyvalent alcohol, and the ester of stearic acid and sorbitol were used as a liquid oil obtained by dissolving in squalane that does not influence male flower buds, branches and leaves to an active ingredient concentration of 5%.

**Table 1**

| | | agent used for immersion test | fatty acid/polyoxyalkylene/ polyvalent alcohol (preparation molar ratio) |
|---|---|---|---|
| Ex. | 1 | polyoxyethylene oleic acid ester | 1/14/0 |
| | 2 | polyoxyethylene linoleic acid ester | 1/6/0 |
| | 3 | methoxypolyoxyethylene oleic acid ester | 1/14/0 |
| | 4 | polyoxyethylene dilinoleic acid ester | 2/14/0 |
| | 5 | polyoxyethylene-block-polyoxypropylene oleic acid ester (5% emulsion) | 1/17/0 (14 mol: oxyethylene, 3 mol: oxypropylene) |
| | 6 | polyoxyethylene sorbitan oleic acid ester | 1/20/1 |
| | 7 | polyoxyethylene sorbitol linoleic acid ester | 1/20/1 |
| | 8 | polyoxyethylene methylglucoside oleic acid ester | 1/20/1 |
| | 9 | polyoxyethylene glycerol linoleic acid ester | 1/20/1 |
| Comp. Ex. | 1 | oleic acid | - |
| | 2 | sodium oleate (5% aqueous solution) | - |
| | 3 | linoleic acid | - |
| | 4 | polyoxyethylene stearic acid ester | 1/14/0 |
| | 5 | polyoxyethylene linolenic acid ester | 1/6/0 |
| | 6 | polyoxyethylene sorbitol | 0/20/1 |
| | 7 | polyoxyethylene sorbitan palmitic acid ester | 1/20/1 |
| | 8 | olive oil | - |
| | 9 | sunflower oil | - |

**Table 2**

| | | fatty acid (or salt thereof) | polyvalent alcohol | fatty acid/polyvalent alcohol (preparation molar ratio) |
|---|---|---|---|---|
| Ex. | 10 | oleic acid | glycerol | 1/1 |
| | 11 | linoleic acid | glycerol | 2/1 |
| | 12 | oleic acid | glycerol | 2/1 |
| | 13 | linoleic acid | glycerol | 1/1 |
| | 14 | linoleic acid | 1,3-butanediol | 1/1 |
| | 15 | oleic acid | pentaerythritol | 3/1 |
| | 16 | oleic acid | neopentylglycol | 1/1 |
| | 17 | linoleic acid | trimethylolpropane | 2/1 |
| | 18 | oleic acid | hexyleneglycol | 1/1 |
| | 19 | linoleic acid | dipropyleneglycol | 1/1 |
| | 20 | linoleic acid | 2,2'-diethyl-2,2'-(oxydimethyl)bis-(propane-1,3-diol) | 2/1 |
| | 21 | oleic acid | diglycerol | 2/1 |
| | 22 | oleic acid | sorbitol | 3/1 |
| | 23 | linoleic acid | glucose | 2/1 |
| | 24 | oleic acid | fructose | 3/1 |
| | 25 | linoleic acid | erythritol | 2/1 |
| | 26 | oleic acid | sucrose | 3/1 |
| | 27 | oleic acid | sorbitol | 2/1 |
| | 28 | oleic acid | sorbitol | 6/1 |
| | 29 | linoleic acid | lactose | 4/1 |
| | 30 | oleic acid | xylitol | 5/1 |
| | 31 | oleic acid | mannitol | 2/1 |
| Comp. Ex. | 10 | stearic acid (5% squalane solution) | diglycerol | 1/1 |
| | 11 | linolenic acid | diglycerol | 1/1 |
| | 12 | oleic acid | diglycerol | 3/1 |
| | 13 | linoleic acid | diglycerol | 3/1 |
| | 14 | stearic acid (5% squalane solution) | pentaerythritol | 3/1 |
| | 15 | palmitic acid (5% squalane solution) | neopentylglycol | 1/1 |
| | 16 | linolenic acid | trimethylolpropane | 2/1 |
| | 17 | linolenic acid | didiglycerol | 2/1 |
| | 18 | stearic acid (5% squalane solution) | sorbitol | 2/1 |
| | 19 | linolenic acid | sorbitol | 2/1 |

**Table 3**

| change of state of male flower bud and needle leaf (August) | | | | |
|---|---|---|---|---|
| | | male flower bud | needle leaf | |
| Example | 1 | browned | no change | |
| | 2 | markedly browned | no change | |
| | 3 | browned | no change | |
| | 4 | markedly browned | no change | |
| | 5 | browned | no change | |
| | 6 | browned | no change | |
| | 7 | browned | no change | |
| | 8 | browned | no change | |
| | 9 | browned | no change | |
| | 10 | markedly browned | no change | |
| | 11 | markedly browned | no change | |
| | 12 | markedly browned | no change | |
| | 13 | markedly browned | no change | |
| | 14 | browned | no change | |
| | 15 | markedly browned | no change | |
| | 16 | browned | no change | |
| | 17 | markedly browned | no change | |
| | 18 | browned | no change | |
| | 19 | browned | no change | |
| | 20 | markedly browned | no change | |
| | 21 | markedly browned | no change | |
| | 22 | markedly browned | no change | |
| | 23 | markedly browned | no change | |
| | 24 | markedly browned | no change | |
| | 25 | markedly browned | no change | |
| | 26 | browned | no change | |
| | 27 | markedly browned | no change | |
| | 28 | browned | no change | |
| | 29 | browned | no change | |
| | 30 | browned | no change | |
| | 31 | markedly browned | no change | |
| Comparative Example | 1 | markedly browned | no change | |
| | 2 | markedly browned | no change | |
| | 3 | markedly browned | no change | |
| | 4 | no change | browned | |
| | 5 | markedly browned | browned | |
| | 6 | no change | no change | |
| | 7 | no change | browned | |
| | 8 | browned | no change | |
| | 9 | markedly browned | no change | |
| | 10 | no change | browned | |
| | 11 | markedly browned | browned | |
| | 12 | browned | no change | |
| | 13 | browned | no change | |
| | 14 | no change | browned | |
| | 15 | no change | browned | |
| | 16 | markedly browned | browned | |
| | 17 | markedly browned | browned | |
| | 18 | browned | browned | |
| | 19 | markedly browned | browned | |

**[Table 4]**

| change of state of male flower bud and needle leaf (November) | | | | |
|---|---|---|---|---|
| | | male flower bud | needle leaf | |
| Example | 1 | browned | no change | |
| | 2 | markedly browned | no change | |
| | 3 | browned | no change | |
| | 4 | markedly browned | no change | |
| | 5 | browned | no change | |
| | 6 | browned | no change | |
| | 7 | browned | no change | |
| | 8 | browned | no change | |
| | 9 | browned | no change | |
| | 10 | markedly browned | no change | |
| | 11 | markedly browned | no change | |
| | 12 | markedly browned | no change | |
| | 13 | markedly browned | no change | |
| | 14 | browned | no change | |
| | 15 | markedly browned | no change | |
| | 16 | browned | no change | |
| | 17 | markedly browned | no change | |
| | 18 | browned | no change | |
| | 19 | browned | no change | |
| | 20 | markedly browned | no change | |
| | 21 | markedly browned | no change | |
| | 22 | markedly browned | no change | |
| | 23 | markedly browned | no change | |
| | 24 | markedly browned | no change | |
| | 25 | markedly browned | no change | |
| | 26 | browned | no change | |
| | 27 | markedly browned | no change | |
| | 28 | browned | no change | |
| | 29 | browned | no change | |
| | 30 | browned | no change | |
| | 31 | markedly browned | no change | |
| Comparative Example | 1 | no change | no change | |
| | 2 | no change | no change | |
| | 3 | no change | no change | |
| | 4 | no change | browned | |
| | 5 | markedly browned | browned | |
| | 6 | no change | no change | |
| | 7 | no change | browned | |
| | 8 | no change | no change | |
| | 9 | no change | no change | |
| | 10 | no change | browned | |
| | 11 | markedly browned | browned | |
| | 12 | no change | no change | |
| | 13 | no change | no change | |
| | 14 | no change | browned | |
| | 15 | no change | browned | |
| | 16 | markedly browned | browned | |
| | 17 | markedly browned | browned | |
| | 18 | no change | browned | |
| | 19 | markedly browned | browned | |

From the results shown in Tables 3 and 4, it was confirmed in the Examples of the present invention that in both August and November, the efficacy on the male flower bud was sharp and needle leaves were not influenced. On the contrary, in Comparative Examples 1, 2 and 3, since oleic acid, sodium oleate and linoleic acid were used as agents, respectively, no effect was obtained for the male flower bud of November.

In Comparative Examples 4 and 7, since stearic acid and palmitic acid derivative was used, no effect was obtained for the male flower buds of both August and November, and the needle leaf was adversely influenced. In Comparative Example 5, since linolenic acid derivative was used as an agent, an effect was obtained for the male flower buds of both August and November, but the needle leaf was adversely influenced. In Comparative Example 6, since fatty acid was not used, the male flower bud and the needle leaf of both August and November were not influenced. In Comparative Examples 8 and 9, since olive oil and sunflower oil were used as agents, respectively, no effect could be obtained for the male flower bud of November.

In Comparative Example 10, since the partial ester of stearic acid and glycerol was used as an agent, no effect was obtained for the male flower bud of both August and November, and the needle leaf was adversely influenced. In Comparative Example 11, since the partial ester of linolenic acid and glycerol was used as an agent, an effect was obtained for the male flower bud in both August and November, but the needle leaf was adversely influenced. In Comparative Example 12, while the ester of oleic acid and glycerol was used as an agent, since 3 hydroxyl groups of glycerol were all esterified with oleic acid, no effect was obtained for the male flower bud of November. In Comparative Example 13, while the ester of linoleic acid and glycerol was used as an agent, since three hydroxyl groups of glycerol were all esterified with linoleic acid, no effect was obtained for the male flower bud of November.

In Comparative Examples 14 and 15, since the ester of stearic acid and palmitic acid and a polyvalent alcohol having a carbon number of 4 or more was used as an agent, no effect was obtained for the male flower bud of both August and November, and the needle leaf was adversely influenced. In Comparative Examples 16 and 17, since the ester of linolenic acid and a polyvalent alcohol having a carbon number of 4 or more was used as an agent, an effect was obtained for the male flower bud of both August and November, but the needle leaf was adversely influenced.

In Comparative Example 18, since the ester of stearic acid and sorbitol was used as an agent, no effect was obtained for the male flower bud of November, and the needle leaf was adversely influenced. In Comparative Example 19, since the ester of linolenic acid and sorbitol was used as an agent, the effect could be obtained for the male flower bud of both August and November, but the needle leaf was adversely influenced.

### Evaluation 2 (evaluation of dilution type agent for suppressing pollen dispersal)

A diluted solution or an emulsion having the formulation shown in Table 5 was prepared, and the effect of each solution for Cryptomeria japonica was evaluated in the same manner as in Evaluation 1. That is, many branches (length about 10 cm) of Cryptomeria japonica with 15 male flower buds on the tip were taken from the same tree of Cryptomeria japonica, and five branches for one group were immersed in the agents (100 ml) of Table 5. After immersing for about 1 min, the branches were taken out from the liquid and placed in a beaker such that the section was immersed in water. Browning changes were inspected visually on the male flower buds and needle leaves after one week. The test was performed using Cryptomeria japonica of August and November. The results are shown in Table 6. The solution was prepared as follows.

### (Preparation method of solution)

The agents in the amounts shown in Table 5 (glycerol was added as stabilizer for emulsification) and water were stirred at 70°C, mixed, treated by a homogenizer (manufactured by MIZUHO Industrial CO., Ltd., QUICK HOMO MIXER LR-1) at 7000 rpm for 3 min, and cooled with stirring.

### (Evaluation of stability of solution)

Each solution was placed in a thermostatic tank which repeated -5°C and 40°C alternately for 12 hr each for 1 month, and the solution state was observed and evaluated as follows.
○: good stability (no change in appearance of solution for 1 month)
x: bad stability (solution separates within 1 month)

### (Evaluation of stability of the solution at high temperature)

Each solution was placed in a thermostatic tank which repeated -5°C and 80°C alternately for 12 hr each for 1 month, and the solution state was observed and evaluated as follows.
○: good stability (no change in appearance of solution for 1 month)
x: bad stability (solution separates within 1 month)

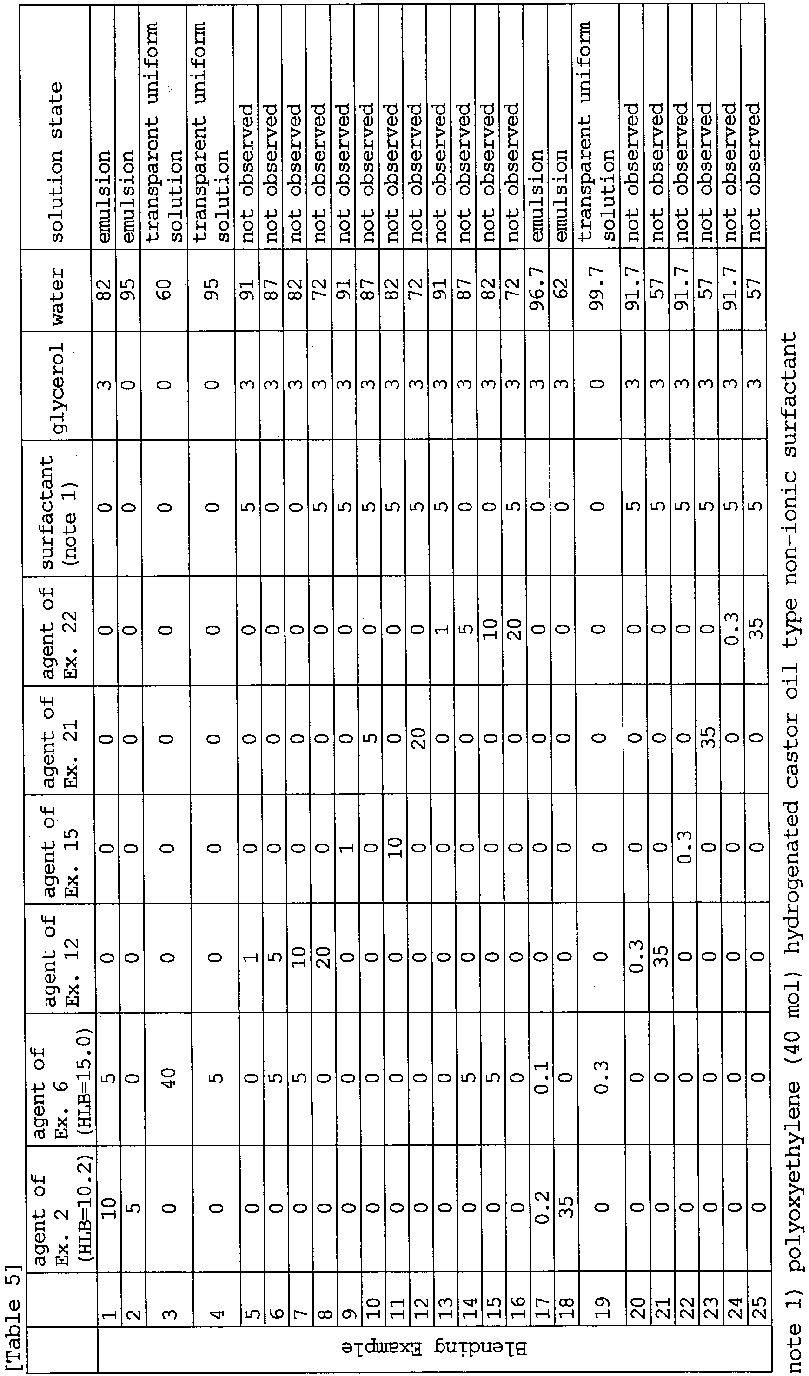

**[Table 6]**

| | August | | November | | stability | stability at high temperature |
|---|---|---|---|---|---|---|
| | male flower bud | needle leaf | male flower bud | needle leaf | | |
| Blending Ex. 1 | markedly browned | no change | markedly browned | no change | ○ | × |
| Blending Ex. 2 | markedly browned | no change | markedly browned | no change | ○ | × |
| Blending Ex. 3 | markedly browned | no change | markedly browned | no change | ○ | ○ |
| Blending Ex. 4 | markedly browned | no change | markedly browned | no change | ○ | ○ |
| Blending Ex. 5 | markedly browned | no change | markedly browned | no change | ○ | not performed |
| Blending Ex. 6 | markedly browned | no change | markedly browned | no change | ○ | not performed |
| Blending Ex. 7 | markedly browned | no change | markedly browned | no change | ○ | not performed |
| Blending Ex. 8 | markedly browned | no change | markedly browned | no change | ○ | not performed |
| Blending Ex. 9 | markedly browned | no change | markedly browned | no change | ○ | not performed |
| Blending Ex. 10 | markedly browned | no change | markedly browned | no change | ○ | not performed |
| Blending Ex. 11 | markedly browned | no change | markedly browned | no change | ○ | not performed |
| Blending Ex. 12 | markedly browned | no change | markedly browned | no change | ○ | not performed |
| Blending Ex. 13 | markedly browned | no change | markedly browned | no change | ○ | not performed |
| Blending Ex. 14 | markedly browned | no change | markedly browned | no change | ○ | not performed |
| Blending Ex. 15 | markedly browned | no change | markedly browned | no change | ○ | not performed |
| Blending Ex. 16 | markedly browned | no change | markedly browned | no change | ○ | not performed |
| Blending Ex. 17 | browned | no change | browned | no change | ○ | × |
| Blending Ex. 18 | markedly browned | no change | markedly browned | no change | × | × |
| Blending Ex. 19 | browned | no change | browned | no change | ○ | ○ |
| Blending Ex. 20 | browned | no change | browned | no change | ○ | not performed |
| Blending Ex. 21 | markedly browned | no change | markedly browned | no change | × | not performed |
| Blending Ex. 22 | browned | no change | browned | no change | ○ | not performed |
| Blending Ex. 23 | markedly browned | no change | markedly browned | no change | × | not performed |
| Blending Ex. 24 | browned | no change | browned | no change | ○ | not performed |
| Blending Ex. 25 | markedly browned | no change | markedly browned | no change | × | not performed |

From the results shown in Table 6, it was confirmed that in both August and November, the efficacy on the male flower bud was sharp and needle leaves were not influenced, in addition a stable diluted solution was obtained. In contrast, in Blending Examples 18, 21, 23 and 25, since the amount of the agent of the Examples to be blended exceeded 30 wt%, the stability of the emulsion was poor.

## Claims

1. Use of an agent comprising, as an active ingredient, an oleic acid derivative or linoleic acid derivative represented by the following formula (I) 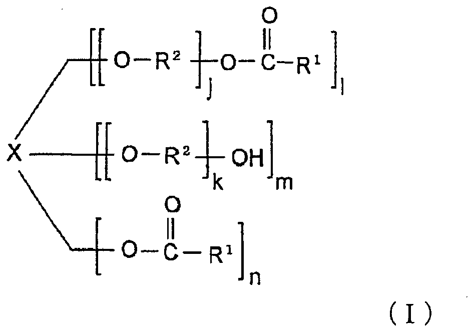 wherein R¹ are residues of an oleic acid ester or a linoleic acid ester, which may be of the same kind or different kinds, O-R² are oxyalkylene groups having a carbon number of 2 to 4, which may be of one kind of two or more kinds, and when they are of two or more kinds, they may be block adducts or random adducts;
when j+k is an integer of one or more and X is monovalent, then X is a hydrogen atom, a hydrocarbon group having a carbon number of 20 or below or an acyl group having a carbon number of 20 or below, j is an integer of 1 - 100, 1=1 and m+n=0;
when j+k is an integer of one or more and X is a polyvalent organic group, then j and k are each an integer of 1 - 100, 1+m+n is an integer of two or more and 1+n is an integer of one or more; when j and k are 0 and X is a polyvalent organic group having a carbon number of 3, then m+n is 3, 1 is 0 and n and m is 1 or 2; and
when j and k are 0 and X is a polyvalent organic group having a carbon number of 4 or more, then 1 is 0, n is an integer of one or more, and m+n is an integer of two or more,
for suppressing pollen dispersal.

2. The use according to claim 1, wherein the oleic acid derivative or linoleic acid derivative is polyoxyethylene oleic acid ester or polyoxyethylene linoleic acid ester, or polyoxyethylene sorbitan oleic acid ester or polyoxyethylene sorbitan linoleic acid ester, or polyoxyethylene sorbitol oleic acid ester or polyoxyethylene sorbitol linoleic acid ester.

3. The use according to claim 2, wherein the agent for suppressing pollen dispersal is an emulsion comprising the oleic acid derivative or linoleic acid derivative, which has an HLB of not more than 12, in a proportion of 0.5 - 30 wt%.

4. The use according to claim 2, wherein the agent for suppressing pollen dispersal is an aqueous solution comprising the oleic acid derivative or linoleic acid derivative, which has an HLB of not less than 12, in a proportion of 0.5 wt% or above.

5. The use according to claim 1, wherein the oleic acid derivative or linoleic acid derivative is comprised of a partial ester of oleic acid or linoleic acid and glycerol.

6. The use according to claim 5, wherein the agent for suppressing pollen dispersal is an emulsion comprising a partial ester of oleic acid or linoleic acid and glycerol in a proportion of 0.5 - 30 wt%.

7. The use according to claim 1, wherein the oleic acid derivative or linoleic acid derivative is comprised of an ester of oleic acid or linoleic acid and a polyvalent alcohol having a carbon number of not less than 4.

8. The use according to claim 7, wherein the agent for suppressing pollen dispersal is an emulsion comprising an ester of oleic acid or linoleic acid and a polyvalent alcohol having a carbon number of not less than 4 in a proportion of 0.5 - 30 wt%.

9. The use according to claim 1, wherein the oleic acid derivative or linoleic acid derivative is comprised of an ester of oleic acid or linoleic acid and an alcohol having a sugar backbone.

10. The use according to claim 9, wherein the agent for suppressing pollen dispersal is an emulsion comprising an ester of oleic acid or linoleic acid and an alcohol having a sugar backbone in a proportion of 0.5 - 30 wt%.

## Patentansprüche

1. Verwendung eines Mittels, das als Wirkstoff ein Ölsäurederivat oder Linolsäurederivat umfasst, welches durch die folgende Formel (I) 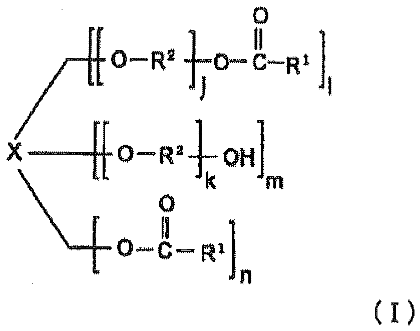 dargestellt wird, wobei R¹ Reste eines Ölsäureesters oder eines Linolsäureesters sind, die gleicher Art oder verschiedener Art sein können, O-R² Oxyalkylengruppen mit einer Kohlenstoffzahl von 2 bis 4 sind, die von gleicher Art oder von zwei oder mehr Arten sein können, und wenn sie von zwei oder mehr Arten sind, können sie Blockaddukte oder statistische Addukte sein;
wenn j+k eine ganze Zahl von eins oder mehr ist und X einwertig ist, ist X ein Wasserstoffatom, eine Kohlenwasserstoffgruppe mit einer Kohlenstoffzahl von 20 oder weniger oder eine Acylgruppe mit einer Kohlenstoffzahl von 20 oder weniger, j ist eine ganze Zahl von 1 bis 100, l = 1 und m+n = 0;
wenn j+k eine ganze Zahl von eins oder mehr ist und X eine mehrwertige organische Gruppe ist, sind j und k jeweils eine ganze Zahl von 1 bis 100, I+m+n ist eine ganze Zahl von zwei oder mehr, und I+n ist eine ganze Zahl von eins oder mehr;
wenn j und k = 0 sind und X eine mehrwertige organische Gruppe mit einer Kohlenstoffzahl von 3 ist, ist m+n = 3, l ist 0, und n und m sind 1 oder 2; und
wenn j und k = 0 sind und X eine mehrwertige organische Gruppe mit einer Kohlenstoffzahl von 4 oder mehr ist, ist l = 0, n ist eine ganze Zahl von eins oder mehr, und m+n ist eine ganze Zahl von zwei oder mehr;
zur Unterdrückung des Pollenflugs.

2. Verwendung gemäß Anspruch 1, wobei das Ölsäurederivat oder Linolsäurederivat Polyoxyethylenölsäureester oder Polyoxyethylenlinolsäureester oder Polyoxyethylensorbitanölsäureester oder Polyoxyethylensorbitanlinolsäureester oder Polyoxyethylensorbitölsäureester oder Polyoxyethylensorbitlinolsäureester ist.

3. Verwendung gemäß Anspruch 2, wobei das Mittel zur Unterdrückung des Pollenflugs eine Emulsion ist, die das Ölsäurederivat oder Linolsäurederivat, das einen HLB-Wert von nicht mehr als 12 aufweist, in einem Anteil von 0,5 bis 30 Gew.-% umfasst.

4. Verwendung gemäß Anspruch 2, wobei das Mittel zur Unterdrückung des Pollenflugs eine wässrige Lösung ist, die das Ölsäurederivat oder Linolsäurederivat, das einen HLB-Wert von nicht weniger als 12 aufweist, in einem Anteil von 0,5 Gew.-% oder mehr umfasst.

5. Verwendung gemäß Anspruch 1, wobei das Ölsäurederivat oder Linolsäurederivat aus einem Partialester von Ölsäure oder Linolsäure und Glycerin besteht.

6. Verwendung gemäß Anspruch 5, wobei das Mittel zur Unterdrückung des Pollenflugs eine Emulsion ist, die einen Partialester von Ölsäure oder Linolsäure und Glycerin in einem Anteil von 0,5 bis 30 Gew.-% umfasst.

7. Verwendung gemäß Anspruch 1, wobei das Ölsäurederivat oder Linolsäurederivat aus einem Ester von Ölsäure oder Linolsäure und einem mehrwertigen Alkohol mit einer Kohlenstoffzahl von nicht weniger als 4 besteht.

8. Verwendung gemäß Anspruch 7, wobei das Mittel zur Unterdrückung des Pollenflugs eine Emulsion ist, die einen Ester von Ölsäure oder Linolsäure und einem mehrwertigen Alkohol mit einer Kohlenstoffzahl von nicht weniger als 4 in einem Anteil von 0,5 bis 30 Gew.-% umfasst.

9. Verwendung gemäß Anspruch 1, wobei das Ölsäurederivat oder Linolsäurederivat aus einem Ester von Ölsäure oder Linolsäure und einem Alkohol mit einem Zuckergerüst besteht.

10. Verwendung gemäß Anspruch 9, wobei das Mittel zur Unterdrückung des Pollenflugs eine Emulsion ist, die einen Ester von Ölsäure oder Linolsäure und einem Alkohol mit einem Zuckergerüst in einem Anteil von 0,5 bis 30 Gew.-% umfasst.

## Revendications

1. Utilisation d'un agent comprenant, comme ingrédient actif, un dérivé d'acide oléique ou un dérivé d'acide linoléique représenté par la formule suivante (I) 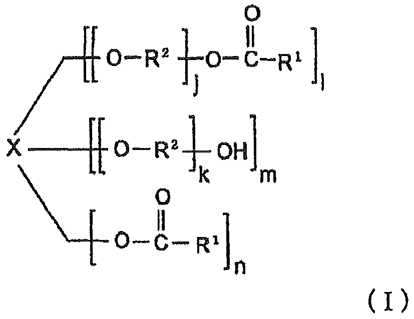 dans laquelle R¹ sont des résidus d'un ester d'acide oléique ou d'un ester d'acide linoléique, qui peuvent être du même type ou de différents types, O-R² sont des groupes oxyalkylène ayant un nombre de carbone de 2 à 4, qui peuvent être d'un type ou de deux types ou plus, et quand ils sont de deux types ou plus, ils peuvent être des produits d'addition blocs ou des produits d'addition aléatoires ;
quand j+k est un entier égal à un ou plus et X est monovalent, alors X est un atome d'hydrogène, un groupe hydrocarbure ayant un nombre de carbone de 20 ou moins ou un groupe acyle ayant un nombre de carbone de 20 ou moins, j est un entier de 1 à 100, 1=1 et m+n=0 ;
quand j+k est un entier égal à un ou plus et X est un groupe organique polyvalent, alors j et k sont chacun un entier de 1 à 100, l+m+n est un entier égal à deux ou plus et 1+n est un entier égal à un ou plus ; quand j et k sont égaux à 0 et X est un groupe organique polyvalent ayant un nombre de carbone de 3, alors m+n est 3, l est égal à 0 et n et m sont égal à 1 ou 2 ; et
quand j et k sont égaux à 0 et X est un groupe organique polyvalent ayant un nombre de carbone de 4 ou plus, alors l est égal à 0, n est un entier égal à un ou plus, et m+n est un entier égal à deux ou plus,
pour supprimer la dispersion du pollen.

2. Utilisation selon la revendication 1, dans laquelle le dérivé d'acide oléique ou le dérivé d'acide linoléique est un ester d'acide oléique polyoxyéthyléné ou un ester d'acide linoléique polyoxyéthyléné ou un ester d'acide oléique de sorbitane polyoxyéthyléné ou un ester d'acide linoléique de sorbitane polyoxyéthyléné ou un ester d'acide oléique de sorbitol polyoxyéthyléné ou un ester d'acide linoléique de sorbitol polyoxyéthyléné.

3. Utilisation selon la revendication 2, dans laquelle l'agent pour la suppression de la dispersion du pollen est une émulsion comprenant le dérivé d'acide oléique ou le dérivé d'acide linoléique, qui a un HLB qui n'est pas supérieur à 12, en une proportion de 0,5 à 30 % en poids.

4. Utilisation selon la revendication 2, dans laquelle l'agent pour la suppression de la dispersion du pollen est une solution aqueuse comprenant le dérivé d'acide oléique ou le dérivé d'acide linoléique, qui a un HLB qui n'est pas supérieur à 12, en une proportion de 0,5 % en poids ou plus.

5. Utilisation selon la revendication 1, dans laquelle le dérivé d'acide oléique ou le dérivé d'acide linoléique comprend un ester partiel d'acide oléique ou d'acide linoléique et de glycérol.

6. Utilisation selon la revendication 5, dans laquelle l'agent pour la suppression de la dispersion du pollen est une émulsion comprenant un ester partiel d'acide oléique ou d'acide linoléique et de glycérol en une proportion de 0,5 à 30 % en poids.

7. Utilisation selon la revendication 1, dans laquelle le dérivé d'acide oléique ou le dérivé d'acide linoléique comprend un ester d'acide oléique ou d'acide linoléique et d'un alcool polyvalent ayant un nombre de carbone qui n'est pas inférieur à 4.

8. Utilisation selon la revendication 7, dans laquelle l'agent pour la suppression de la dispersion du pollen est une émulsion comprenant un ester d'acide oléique ou d'acide linoléique et d'un alcool polyvalent ayant un nombre de carbone qui n'est pas inférieur à 4 en une proportion de 0,5 à 30 % en poids.

9. Utilisation selon la revendication 1, dans laquelle le dérivé d'acide oléique ou le dérivé d'acide linoléique comprend un ester d'acide oléique ou d'acide linoléique et d'un alcool ayant un squelette de sucre.

10. Utilisation selon la revendication 9, dans laquelle l'agent pour la suppression de la dispersion du pollen est une émulsion comprenant un ester d'acide oléique ou d'acide linoléique et d'un alcool ayant un squelette de sucre en une proportion de 0,5 à 30 % en poids.
